Europäisches Patentamt

**European Patent Office**

Office européen des brevets

(11) Publication number: **0 085 958**
A2

(12) **EUROPEAN PATENT APPLICATION**

(21) Application number: 83101033.5

(22) Date of filing: 03.02.83

(51) Int. Cl.³: **C 12 P 13/22**
C 12 N 15/00, C 12 N 1/20
//C12R1/125

(30) Priority: 05.02.82 JP 17089/82

(43) Date of publication of application:
17.08.83 Bulletin 83/33

(84) Designated Contracting States:
DE FR GB

(71) Applicant: AJINOMOTO CO., INC.
5-8, Kyobashi 1-chome, Chuo-ku
Tokyo 104(JP)

(72) Inventor: Kurahashi, Osamu
No. 958, Kashimada Saiwai-ku
Kawasaki-shi Kanagawa-ken(JP)

(72) Inventor: Tsuchida, Takayasu
No. 1730-13, Kamikurata-cho Totsuka-ku
Yokohama-shi Kanagawa-ken(JP)

(72) Inventor: Kawashima, Hiroki
No. 2-20-8, Kannon Kawasaki-ku
Kawasaki-shi Kanagawa-ken(JP)

(72) Inventor: Enei, Hitoshi
No. 2-30-2, Ikego
Zushi-shi Kanagawa-ken(JP)

(74) Representative: Schübel-Hopf, Ursula et al,
Strehl, Schübel-Hopf, Schulz Patentanwälte
Widenmayerstrasse 17
D-8000 München 22(DE)

(54) Method for producing l-phenylalanine by fermentation.

(57) L-phenylalanine producing microorganisms, which have been constructed by introducing a recombinant plasmid DNA inserted with a chromosomal DNA fragment controlling the resistance to a phenylalanine-antagonist obtained from a mutant of the genus *Bacillus* resistant to the phenylalanine antagonist, into a recipient strain of the genus *Bacillus*, produce L-phenylalanine in higher yield than the mutants used as the DNA-donor or the recipient strain.

EP 0 085 958 A2

# METHOD FOR PRODUCING L-PHENYLALANINE BY FERMENTATION

The present invention relates to a method for producing L-phenylalanine by fermentation, and particularly to a method for producing L-phenylalanine with a microorganism of the genus Bacillus constructed by a gene splicing technique.

In order to render a wild strain capable of producing L-phenylalanine by fermentation, it has been necessary in the past to induce artificial mutants from the wild strain. In this regard, there are many known L-phenylalanine producing artificial mutants.

Examples of known phenylalanine producing microorganisms include mutants of Bacillus subtilis (J. Biol. Chem., 242, 4948 (1967), mutants of Corynebacterium or Brevibacterium resistant to phenylalanine-analogues(U.S. Patent No. 3,660,235), mutants of Corynebacterium resistant to phenylalanine-analogues and requiring L-tyrosine for growth, and mutants of Brevibacterium resistant to phenylalanine-analogues and tryptophan-analogues and requiring L-tyrosine for growth (U.S. Patent No. 3,909,353).

Another approach to increase the productivity of phenylalanine in microorganisms is suggested in case of Escherichia in Japanese Published Unexamined Patent Application No. 165798/1979), in which Escherichia coli strains transformed with a recombinant plasmid DNA and constructed by a gene splicing technique to produce L-phenylalanine, are disclosed.

However, it is still requested to produce L-phenylalanine by fermentation in higher efficiency than the known methods.

The problem to be solved by the present invention is therefore the production of new microorganism strains which are able to produce L-phenylalanine in higher yields than the known L-phenylalanine producing microorganism strains.

It has now been found that the L-phenylalanine producing microorganisms, which have been constructed by introducing a recombinant plasmid DNA inserted with a chromosomal DNA fragment con trolling the resistance to a phenylalanine-antagonist obtained from a mutant of the genus Bacillus resistant to the phenylalanine antagonist, into a recipient strain of the genus Bacillus, produce L-phenylalanine in higher yield than the mutants used as the DNA-donor or the recipient strain.

In the following the invention is described in more detail by means of preferred embodiments.

The L-phenylalanine producing microorganisms used in the method of the present invention are constructed by introducing a recombinant plasmid DNA inserted with a chromosomal DNA fragment controlling the resistance to a phenylalanine-antagonist obtained from a mutant of the genus Bacillus resistant to the phenylalanine-antagonist, into a recipient strain of the genus Bacillus.

The phenylalanine-antagonists of the present invention inhibit the growth of Bacillus, but the inhibition is suppressed partially or completely when L-phenylalanine coexists in the medium. Examples of the L-phenylalanine-antagonists are o, m or p-fluorophenylalanine, o, m or p - aminophenylalanine, β-phenylserine, cyclohexylserine, α-amino-β-phenylethanesulfonic acid, o, m or p - bromophenylalanine, β-2-thienylalanine, β-3-thienylalanine, 1-cyclo-phentene -1-alanine, 1-cyclohexene-1-alanine, 2-amino-4-methyl-hexenic acid, S-(1, 2-dichlorovinyl)-cysteine, β-4-pyridylalanine, β-2-pyridylalanine, β-4-pyrazolealanine and p-nitro-phenylalanine.

Although any mutant of the genus Bacillus resistant to phenylalanine-antagonists can be used as the DNA-donor for the chromosomal DNA fragment controlling the resistance to the phenylalanine-antagonists, mutants having higher resistance to the phenylalanine-antagonists are preferred. In many cases, better results can be obtained when a mutant having higher productivity of L-phenylalanine is used as the DNA-donor. The mutant resist-ant to the phenylalanine-antagonists can be obtained by a conventional manner such as exposing a parent strain to 250 μg/mℓ of N-methyl-N'-nitro-N-nitrosoguanidine, and isolating the strain capable of growing in a medium containing an amount of the phenylalanine-antagonist which inhibits the growth of the parent strain.

The extraction of chromosomal DNA can be carried out by a conventional method as described in Bacteriol, 89, 1065, (1965).

As the vector DNA, plasmid DNAs which propagate in hosts of Bacillus are used. Typical vector DNAs are pCT 127, pC 194, pC 221, pC 223 and pUB 112 (Proc. Natl, Acad, Sci. U.S.A., 74, 1680-1682 (1977)), pUB 110 (J. Bacteriol., 134, 318-329 (1978), and pTP 4 and pTP 5 (Microbiol Letters, 5, 55-59 (1978)), all of which are derived from plasmids of Staphylococcus, and pLS 15 and pLS 28 (J. Bacteriol., 131, 699-701 (1977)), pLS 13 (J. Bacteriol., 129, 1487-1494 (1977)), and pPL, pPL 2 (J. Bacteriol 124, 484 (1975), all of which are derived from plasmids of Bacillus.

The chromosomal DNA is digested with a restriction endonuclease by a well known method (Biochem. Biophys. Acta, 383, 457, (1975)). Various kind of restriction endonucleases can be used if the degree of digestion is controlled by appropriately selecting the reaction time.

The vector DNA is also cleaved with a restriction endonuclease. Suitable restriction endonucleases for each vector DNA are disclosed in the literature shown in the parenthesis above.

The recombination of DNA to prepare the recombinant plasmid can be carried out by the ligation reaction with a ligase, or by incorporating with terminal transferase deoxyadenylic acid and thymidylic acid, or deoxyguanylic acid and deoxycytidylic acid into the chromosomal DNA fragment and cleaved vector DNA and by subjecting the modified chromosomal DNA fragment and cleaved DNA to an annealing reaction.

The recombinant DNA thus obtained can be incorporated into the DNA-recipient by treating the cells of the DNA-recipient with calcium chloride to increase the permeability as is reported regarding E. coli K-12 (Mandel, M. and Higa, A., J. Mol. Biol., 53, 159 (1970)), or by applying for the incorporation cells of the DNA-recipient at a specific stage of growth when the cells become capable of incorporating plasmids (competent cells) as is reported for Bacillus subtilis (Duncan, C.H., Wilson, G.A. and Young, F.E., Gene 1, 153 (1977)). The recombinant DNA can also be incorporated into the DNA-recipient by forming a protoplast or spheroplast of the DNA-recipient which easily incorporates plasmid DNA as is known for Bacillus subtilis, actinomycetes and yeast (Chang, S. and Cohen, S.N., Molec, Gen. Genet, 168, 111 (1979)); Bibb, M.J. Ward, J.M. and Hopwood, O.A., Nature, 274, 398 (1978); Hinnen, A., Hicks, J.B. and Fink, G.R., Proc. Natl, Acad. Sci., USA, 75, 1929 (1978)).

The recipients for the recombinant DNA are microorganisms of the genus Bacillus. It is convenient to use, as the DNA recipient, microorganisms sensitive to phenylalanine-analogue and requiring histidine for growth for the selection of a transformant having a recombinant DNA inserted with chromosomal DNA fragment controlling resistance to phenylalanine-antagonist. When the recombinant plasmid DNA inserted with chromosomal DNA fragment controlling the resistance to phenylalanine-antagonist is used for transformation after selection of the recombinant plasmid DNA using a host sensitive to phenylalanine-antagonist and requiring L-phenylalanine for growth, microorganisms resistant to phenylalanine-antagonist and having no requirement of L-phenylalanine for growth can be used as the DNA recipient.

The desired transformants are those which have become resistant to phenylalanine-antagonist and are capable of producing L-phenylalanine in case microorganisms sensitive to phenylalanine-antagonists and requiring L-phenylalanine for growth are used as the recipients. In case of microorganisms resistant to phenylalanine-antagonists and having no requirement of L-phenylalanine for growth, the desired transformants are those which have the characteristics possessed by the vector DNA as the marker (which may be resistance to one or more drugs) for selection.

The methods of cultivation of the L-phenylanine producing transformants thus obtained are conventional, and are similar to the methods for the cultivation of known L-phenylanine producing microorganisms. The aqueous culture medium employed is a conventional one containing a carbon source, nitrogen source, inorganic ions and, when required, minor organic nutrients such as vitamines and amino acids.

As to the carbon source, carbohydrates such as glucose, sucrose, lactose, fructose and raw material containing such saccharides (such as strach hydrolysate, molasses and fruit juice) are used. Gaseous ammonia, aqueous ammonia, ammonium salts and other nitrogen containing materials can be used as the nitrogen source.

The cultivation is conducted under an aerobic condition in which the pH and the temperature of the aqueous culture medium are adjusted to a suitable level previously determined and continued until the production of L-phenylalanine cases.

Example 1

(1)  Extraction of chromosomal DNA

Bacillus subtilis AJ 11773 (FERM-P 6319 = FERM-BP 237) requiring L-arginine and L-leucine and resistant to DL-m-fluorophenylalanine was cultured in 1 ℓ of "Bact Penassay Broth" (Difco) at 30°C for 2 hours with shaking, and cells in exponential growth phase were harvested.  Chromosomal DNA was extracted from the cells by a conventional phenol-method, (J. Bacteriol., 89, 1065 (1965), and 3.8 mg of purified DNA was obtained.

(2)  Insertion of chromosomal DNA fragment into vector

As the vector, pUB 110 possessing the genetic information for kanamycin-resistance and neomycin-resistance was used.

Ten μg of the chromosomal DNA obtained in step (1) and 5 μg of the vector DNA were digested separately with endonuclease Eco RI at 37°C for 1 hour, and thereafter the two reaction mixtures were heated at 65°C for 10 minutes and mixed.  The mixed solution was subjected to ligation reaction by a T₄ phage DNA-ligase in the presence of ATP and dithreitol at 10°C for 24 hours.

(3)    Genetic transformation with the plasmid having phenylalanine producing gene.

Bacillus subtilis AJ 11772 (FERM-P 6318 = FERM-BP 236)which requires L-arginine, L-leucine and L-phenylalanine was cultured in "Penassay Broth" (Difco) at 30°C overnight with shaking, and thereafter culturing at 37°C for 4 hours with shaking in Medium-I (containing 0.5 g/dl glucose, 0.2 g/dl $(NH_4)_2SO_4$, 0.6 g/dl $KH_2PO_4$, 1.4 g/dl $K_2HPO_4$, 0.02 g/dl $MgSO_4$. $7H_2O$, 0.1 g/dl sodium citrate, 0.2 g/dl yeast extract, 5 mg/dl L-phenylalanine, 25 mg/dl L-arginine and 5 mg/dl L-leucine), and further cultured, after the cultivation in Medium-I, at 37°C for 1.5 hours with shaking in Medium-II (containing 0.5 g/dl glucose, 0.2 g/dl $(NH_4)_2SO_4$, 0.6 g/dl $KH_2PO_4$, 1.4 g/dl $K_2HPO_4$, 0.12 g/dl $MgSO_4 \cdot 7H_2O$, 0.1 g/dl sodium citrate, 0.02 g/dl yeast extract, 5 mg/dl L-arginine and 0.5 mg/dl L-leucine).  Thus, competent cells having the ability of plasmid uptake were obtained.  (C. Anagnostopoulos, J. Spizizen: J. Bacteriol., 81, 741, (1961)).

A suspension of the competent cells was added with the recombinant plasmid obtained in step (2), and incubated at 37°C for 2 hours with shaking to complete the transformation reaction.

The cell-suspension was transferred onto a minimum medium prepared by adding 5 μg/dl kanamycin, 10 mg/dl L-leucine, 10 mg/dl L-leucine, 10 mg/dl L-arginine, 100 mg/dl D, L-m-fluorophenyla-lanine and 2 g/dl agar to a basal minimum medium of pH 7.2 containing 0.6 g/dl $KH_2PO_4$, 1.4 g/dl $K_2HPO_4$, 0.2 g/dl $(NH_4)_2SO_4$, 0.1 g/dl sodium citrate, 0.02 g/dl $MgSO_4 \cdot 7H_2O$, and 0.5 g/dl

glucose. After 3 days cultivation at 37°C, two colonies appeared on the agar-medium.

Among the transformants, AJ 11774 (FERM-P 6320 = FERM-BP 238) which had highest productivity of L-phenylalanine was selected. DNA in AJ 11774 was extracted from it by C.I. Kado's phenol method (J. Bac., 145, 3, 1365 (1981)). Plasmid DNA and chromosomal DNA were separated by agarose-gel electrophoresis, and plasmid DNA obtained was purified by dialysis.

The purified plasmid DNA was incorporated into AJ 11773, which produces L-phenylalanine, by the manner shown in step (3), and as the desired transformant, kanamycin-resistant AJ 11775 (FERM-P 6321 = FERM-BP 239)was obtained.

(4)  Phenylalanine production by the new phenylalanine producers L-phenylalanine productivity of AJ 11773, AJ 11774 and AJ 11775 were tested as follows.

Twenty ml batches of a culture medium at pH 7.0, which contained, per deciliter, 8 g glucose, 1 g $NH_4Cl$, 0.2 g KCl, 0.1 g $KH_2PO_4$, 0.04 g $MgSO_4 \cdot 7H_2O$ , 0.4 g "casamino acid" (Difco), 1 mg $FeSO_4 \cdot 4H_2O$, 1 mg $MnSO_4 \cdot 4H_2O$, 20 mg L-arginine, 20 mg L-leucine and 4 g $CaCO_3$, were placed in 500 ml-shaking flasks. Five µg/ml kanamycin was added further to the medium for AJ 11774 and AJ 11775.

The cultivation was carried out at 30°C for 96 hours with shaking.

The amounts of L-phenylalanine in the supernatant of the resulting culture media were determined, and are shown in Table 1.

Table 1

| Microorganism tested | L-phenylalanine accumulated (mg/d ) |
|---|---|
| AJ 11773 (FERM BP 237) | 162 |
| AJ 11774 (FERM BP 238) | 185 |
| AJ 11775 (FERM BP 239) | 288 |

Bacillus subtilis AJ 11772, Bacillus subtilis AJ 11773, Bacillus subtillis AJ 11774 and Bacillus subtilis AJ 11775 have the taxonomic characteristics as disclosed in Proc. Sec. Int. Congr. Microbiol. (London, 1936) 245 (1937) and Nomencl. Comm. Intern. Soc. Microbiol., 28, (1937), and in addition have characteristics as disclosed hereinabove.

Bacillus subtilis AJ 11772, AJ 11773, AJ 11774 and AJ 11775 were deposited at the Fermentation Research Institute, Japan, on the January 27, 1982 under the accession numbers FERM-P 6318, FERM-P 6319, FERM-P 6320 and FERM-P 6321, respectively, which deposits were converted to deposits under the Budapest Treaty on January, 1983.

CLAIMS :

1.   A method for producing L-phenylalanine by fermentation which comprises aerobically culturing an L-phenylalanine producing microorganism in an aqueous culture medium, and recovering the L-phenylalanine accumulated in the culture medium, characterized in that said L-phenylalanine producing microorganism has been constructed by incorporating a recombinant plasmid DNA inserted with a DNA fragment controlling the resistance to a phenylalanine-antagonist, obtained from a chromosomal DNA of a mutant of the genus Bacillus resistant to the phenylalanine-antagonist, into a recipient strain of the genus Bacillus.

2.   The method according to claim 1, wherein said mutant belongs to Bacillus subtilis.

3.   The method according to claim 1 or 2, wherein said recipient strain belongs to Bacillus subtilis.

4.   The method according to any of the claims 1 to 3, wherein said phenylalanine-antagonist is m-fluorophenylalanine.

5.   The method according to claim 1 or 3, wherein said recipient strain requires L-phenylalanine for growth.

6. The method according to any of the claims 1 to 5, wherein said recipient strain is resistant to a phenylalanine-antagonist.

7. A method for producing L-histidine by fermentation which comprises :

aerobically culturing in a culture medium an L-phenylalanine producing microorganism which is produced by incorporating a first recombinant plasmid into a first recipient strain of the genus Bacillus, said first recombinant plasmid containing a DNA fragment controlling the resistance to a phenylalanine-antagonist, obtained from a transformant of the genus Bacillus which is produced by incorporating a second hybrid plasmid into a second recipient strain of the genus Bacillus, said second recombinant plasmid containing a DNA fragment controlling the resistance to a phenylalanine-antagonist, obtained from a mutant of the genus Bacillus resistant to a phenylalanine-antagonist, said second recipient strain of the genus Bacillus being an L-phenylalanine requiring strain, and said first recipient strain of the genus Bacillus being resistant to a phenylalanine-antagonist.

8. Bacillus subtilis FERM BP 238 und FERM BP 239 and their artificial mutants induced in a manner known per se and their transformants obtained by gene technology in a manner known per se which are capable of producing L-phenylalanine.